# FASCICULE DE BREVET EUROPEEN

(11) **EP 1 061 897 B1**
(45) Date de publication et mention de la délivrance du brevet: **25.09.2002**
(21) Numéro de dépôt: 99909034.3
(22) Date de dépôt: 18.03.1999
(51) Int. Cl.: A61K 7/48, A61K 38/05, A23J 3/00

(54) **OLEAMIDE DE GLYCYLGLYCINE EN DERMO-COSMETOLOGIE**
OLEAMID-GLYCYL-GLYCINE DERIVATE FÜR DIE KOSMETIK UND/ODER DERMATOLOGIE
GLYCYLGLYCINE OLEAMIDE IN DERMO-COSMETOLOGY

(30) Priorité: 20.03.1998 FR 9803449
(43) Date de publication de la demande: 27.12.2000
(73) Titulaire: Pierre Fabre Dermo-Cosmetique, 92100 Boulogne-Billancourt (FR)
(72) Inventeur: FORT LACOSTE, Lydie, F-31120 Roquettes (FR); BEL, Agnès, F-81500 Lavaur (FR); GINESTAR-GONZALES, José, F-31400 Toulouse (FR); NAVARRO, Roger, F-09100 Pamiers (FR)
(74) Mandataire: Ahner, Francis
(86) Numéro de dépôt international: FR9900621
(87) Numéro de publication internationale: WO99048470

(56) Documents cités:
- EP-A- 0 538 764
- WO-A-96/28008
- FR-A- 2 666 226
- FR-A- 2 668 365
- US-A- 4 732 690
- DATABASE WPI Week 8426 Derwent Publications Ltd., London, GB; AN 84-161105 XP002091813 & JP 59 084994 A (KAWAKEN FINE CHEM CO. LTD), 16 mai 1984 (1984-05-16)

## Description

La présente invention concerne l'utilisation de l'oléamide de glycylglycine en dermo-cosmétologie pour fabriquer une composition destinée à protéger les fibres de soutien du tissu conjonctif des phénomènes de glycation et d'élastose, et d'une manière plus générale en stimulant le métabolisme cellulaire.

Parallèlement au vieillissement cutané intrinsèque, dit chronologique, qui est génétiquement programmé, il existe un vieillissement cutané induit, où différents facteurs extrinsèques participent, parmi lesquels les U.V. et le stress oxydatif jouent un rôle important.

Au niveau du derme, on observe donc, à côté d'une diminution de l'activité métabolique du fibroblaste, une altération des fibres de la matrice, notamment des fibres de collagène et d'élastine.

Ainsi, les fibres de collagène sont particulièrement sensibles à la glycation qui est un des phénomènes responsables du vieillissement des structures protéiques (M. STERNBERG, P. URIOS, A. BORSOS, G. MOZERE, G. HIRBEC, R. GUILLOT, J. PEYROUX, J. Med. Esth. et Chir. Derm., Mars 97 (93), XXIV, 13-18). Il s'agit d'une réaction non enzymatique entre un sucre réducteur, comme le glucose, et une protéine à longue durée de vie, comme le collagène. Elle se déroule en deux étapes :
- réaction entre la fonction aldéhyde d'un sucre réducteur et la fonction amine d'une protéine : formation d'une base de Schiff qui subit un réarrangement en produit d'Amadori. Cette étape est réversible, puis
- intervention de processus oxydatifs au cours desquels se forment des produits intermédiaires très réactifs, des déoxyosones. Ces derniers possèdent des fonctions cétones qui leur permettent de réagir avec d'autres fonctions amines libres portées par d'autres protéines. On aboutit ainsi à un pontage irréversible des fibres avec formation de produits finaux de glycation (A.G.E. en abrégé), dont le taux augmente avec l'âge. On observe une réticulation des fibres de collagène, entraînant probablement une altération des interactions fibroblaste/matrice si bien que le tissu conjonctif se sclérose.

La raréfaction des fibres élastiques est un autre phénomène observé au cours du vieillissement (L. ROBERT, A.M. ROBERT, J. Med. Esth. Et Chir. Derm., 1993 (12), XX, (80), 211-217).

Dès l'âge adulte, la synthèse d'élastine devient plus faible et très lente. A ce phénomène s'ajoute une destruction accrue de l'élastine par des élastases, enzymes protéolytiques dont l'activité augmente avec l'âge.

Par ailleurs, l'exposition chronique aux U.V, provoque des altérations qui viennent s'ajouter au vieillissement intrinsèque. Les fibres élastiques, sous l'effet des U.V., deviennent plus courtes et plus épaisses, elles se transforment en une masse amorphe: on parle d'élastose actinique.

Les élastases relèvent apparemment de plusieurs catégories d'enzymes. La plus importante paraît être l'élastase produite par les fibroblastes cutanés eux-mêmes.

Une autre source potentielle d'élastases est représentée par les leucocytes véhiculés par le sang. Les leucocytes polynucléaires portent dans leurs granules une sérine-protéase puissante (l'élastase leucocytaire) pouvant attaquer non seulement les fibres élastiques mais pratiquement tous les constituants de la matrice extra-cellulaire.

Cette enzyme est particulièrement activée dans des phénomènes inflammatoires, qui peuvent notamment être observés après une exposition aux U.V.

Pour lutter contre les mécanismes impliqués dans le vieillissement intrinsèque et/ou extrinsèque que nous avons décrits précédemment, de nombreuses voies d'approche sont proposées.

La demande de brevet FR-2 666 226 propose d'associer un système filtrant sur l'étendue du spectre des fréquences photoniques, au moins un composant anti-radicalaire comme la vitamine E et, au moins, un composant anti-élastasique, comme la vitamine P.

Par ailleurs, la demande de brevet FR-2 668 365 décrit des acylpeptides pour lutter contre le vieillissement. Plus précisément, ce document démontre que la carnosine ou le peptide Gly-His-Lys, chacun acylé du côté N-terminal par un acide gras en C12-C18, conservent respectivement leur activité biologique antioxydante ou stimulatrice de la synthèse de coltagène. Dans les compositions cosmétiques décrites, l'acide gras confère un caractère lipophile plus marqué au peptide actif pour faciliter sa pénétration dans le stratum corneum. L'acide gras en question ne sert donc qu'à véhiculer le peptide au niveau cutané et ne présente aucune activité biologique propre.

La demande de brevet WO-96/28008 propose d'associer dans un produit anti-vieillissement deux principes actifs : un actif capable d'inhiber la formation du produit d'Amadori dès la première étape de glycation du collagène, et un actif ayant des propriétés anti-élastase, de manière à protéger les deux types de fibres du tissu conjonctif. WO-96/28008 porte donc sur l'association de deux actifs pour lutter contre le vieillissement cutané.

L'oléamide de glycylglycine de formule :

CH₃―(CH₂)₇―CH=CH―(CH₂)₇―CO―NH―CH₂―CO―NH―CH₂―COOH

est un lipo-peptide ou acyl-peptide issu de la condensation de l'acide oléique et du dipeptide glycylglycine.

L'oléamide de glycylglycine est connu pour ses propriétés tensioactives dans des compositions détergentes liquides (US-4 732 690 et JP-59 84 994).

La présente invention, a pour objet l'utilisation de l'oléamide de glycylglycine pour fabriquer une composition destinée à lutter contre la glycation et l'élastose des fibres protéiques du tissu conjonctif,

Plus précisément, la composition contenant l'oiéamide de glycylglycine selon l'invention permet, d'une part, de protéger le collagène des phénomènes précoces de glycation, et d'autre part, de protéger l'élastine de l'attaque des élastases, tout en assurant -et ceci de manière tout à fait novatrice- une action sur le métabolisme du fibroblaste.

Dans le cadre de la présente invention, la composition contenant l'oléamide de glycylglycine permet de lutter contre tous les processus biologiques dans lesquels sont impliqués la glycation et/ou l'élastose des protéines.

La composition contenant l'oléamide de glycylglycine permet avantageusement de lutter contre le vieillissement cutané photo-induit et de stimuler le métabolisme cellulaire pour améliorer la qualité de la peau et des phanères.

La composition contenant l'oléamide de glycylglycine permet également de lutter contre les complications du diabète.

La composition contenant l'oléamide de glycylglycine est de préférence une composition cosmétique, dermatologique ou alimentaire.

La composition décrite précédemment contient de préférence 0,0001 à 20 % en poids d'oléamide de glycylglycine par rapport au poids total de la composition.

Cette composition contient avantageusement un agent anti-glyquant comme l'amino-guanidine, la glycylglycine ou le bromure de phénacylthiazolium, et/ou de l'acide oléique.

La composition selon l'invention peut également contenir tout excipient approprié et d'autres agents actifs tels que les agents anti-vieillissement, comme la DHEA ; les rétinoïdes, notamment le rétinal ; des vasculoprotecteurs, comme la rutine, l'hespéridylméthylchalcone ; des anti-radicalaires, des chélateurs du fer, comme le furildioxime ; des hormones, telles que les oestrogènes ; des vitamines, dont la vitamine C ; des anti-inflammatoires ; des dépigmentants, dont l'hydroquinone ; des anti-chutes, dont le minoxidil ; des agents hydratants dont le glycolate de guanidine ; des filtres solaires ; des cicatrisants ; des eaux thermales ; au moins un hydrolysat de protéine aux propriétés chélatrices ; un agent de protection contre les UV, en particulier un écran minéral.

Les exemples suivants illustrent l'invention sans en limiter la portée, et proposent notamment un procédé de synthèse de l'oléamide de glycylglycine et des compositions derme-cosmétiques contenant l'oléamide de glycylglycine.

La figure 1 représente l'activité antlyquante (exprimée en nmoles HMF/mg d'hydrolysat) d'un actif anti-glyquant classique, l'aminoguanidine, et l'activité antiglyquante de la glycylglycine (gly-gly) avec un effet dose (1 mM, 10 mM et 100 mM).

La figure 2 donne l'activité anti-élastase in vitro (exprimée par l'aire de lyse en cm² sur un gel d'argose) du mélange glycylglycine (gly-gly), de l'acide oléique, du mélange glycylglycine/acide oléique, et de l'oléamide de glycylglycine.

La figure 3 donne la cinétique de rétraction in vitro de six structures tissulaires tridimensionnelles contenant respectivement 0,002 % de gly-gly, 0,003 % d'acide oléique, 1 % ou 5 % d'un témoin, 0,003 % d'acide oléique, un mélange de 0,002 % de gly-gly et 0,005 % d'oléamide de glycylglycine. On donne en ordonnée le pourcentage de rétraction et en abscisse le temps en heures.

### EXEMPLE 1 : Préparation de l'oléamide de glycylglycine

Dans un ballon, sous agitation magnétique, la glycylglycine (1 eq) est mise en suspension dans 60 ml de tétrahydrofurane et 10 ml d'eau. On y ajoute la soude microperlée (1,3 eq) puis on agite à température ambiante jusqu'à dissolution complète. On coule ensuite goutte à goutte l'oléoylchlorure (1,3 eq). On laisse le mélange 12 heures à température ambiante sans agitation, puis on le dilue avec 100 ml d'eau. On filtre le précipité. Les cristaux obtenus sont blancs crème d'aspect gras. On réempâte les cristaux dans 60 ml de méthanol à reflux (65°C) pendant 1 heure. On refroidit à 15 ± 5°C et on filtre. Les cristaux blanc crème sont séchés sous étuve à vide à 50°C. Le rendement est de 50 %.

On peut également utiliser comme source d'acides gras, des hydrolysats d'huiles riches en acide oléïque, et comme source de glycylglycine, des hydrolysats de protéines.

### EXEMPLE 2 : Mise en évidence in vitro de l'activité antiglyquante de la glycylglycine vis-à-vis des phénomènes précoces de glycation

L'activité inhibitrice de la glycylglycine est évaluée et comparée à celle de l'aminoguanidine en utilisant un modèle in vitro, acellulaire, de glycation du collagène de type I, selon le protocole suivant.

### 1) Glycation in vitro du collagène

L'actif anti-glyquant est introduit dans une solution contenant du glucose 250 mM et du collagène de type I. L'aminoguanidine agit notamment en bloquant le groupement carbonyle du produit d'Amadori, stoppant ainsi la glycation à son stade précoce (M. Brownlee et coll., *Science,* 1986 (6), 232, 1629-1632).

Le mélange est incubé à 37°C pendant 8 jours puis le glucose non fixé est éliminé par dialyse. Après lyophilisation du collagène, on procède à un dosage à l'acide thiobarbiturique pour déterminer le taux de glycation.

### 2) Dosage à l'acide thiobarbiturique

Cette méthode permet le dosage de sucres liés aux cétoamines.

Le lyophilisat, après être pesé, est dissous dans l'acide oxalique 0,5 M. On laisse incuber à 120°C pendant 1 heure, puis à 40°C pendant 45 min après avoir ajouté de l'acide thiobarbiturique à 50 mM.

La densité optique du mélange, lue à 443 nm, est proportionnelle au taux de glycation exprimé en nmoles HMF formé par mg de lyophilisat.

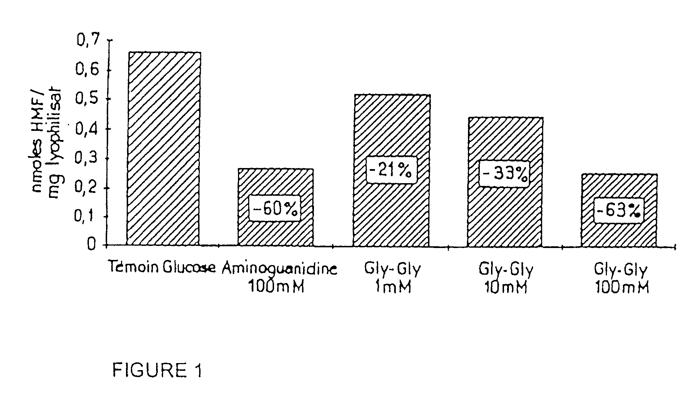

Les résultats obtenus présentés figure 1 montrent l'activité anti-glyquante de la glycylglycine avec un effet-dose. A 100 mM, on obtient une activité équivalent à celle de l'aminoguanidine, actif anti-glyquant de référence.

### EXEMPLE 3 : Mise en évidence in vitro de l'activité anti-élastase de l'oléamide de glycylglycine et de l'acide oléique.

L'activité anti-élastase de l'oléamide de glycylglycine est évaluée comparativement à celle de l'acide oléïque seul, la glycylglycine seule et de l'acide oléique associé à la glycylglycine (B. Ashe et coll., *Biochem. Biophys. Res. Commun,* 1977, 75, 194).

La méthode utilisée est une technique in vitro acellulaire, basée sur la diffusion d'élastase dans un gel d'agarose contenant de l'élastine insoluble colorée par l'orcéïne et dispersée dans le gel avec ou sans principe actif. La lecture du diamètre de lyse permet de calculer le pourcentage d'inhibition de l'enzyme par le principe actif.

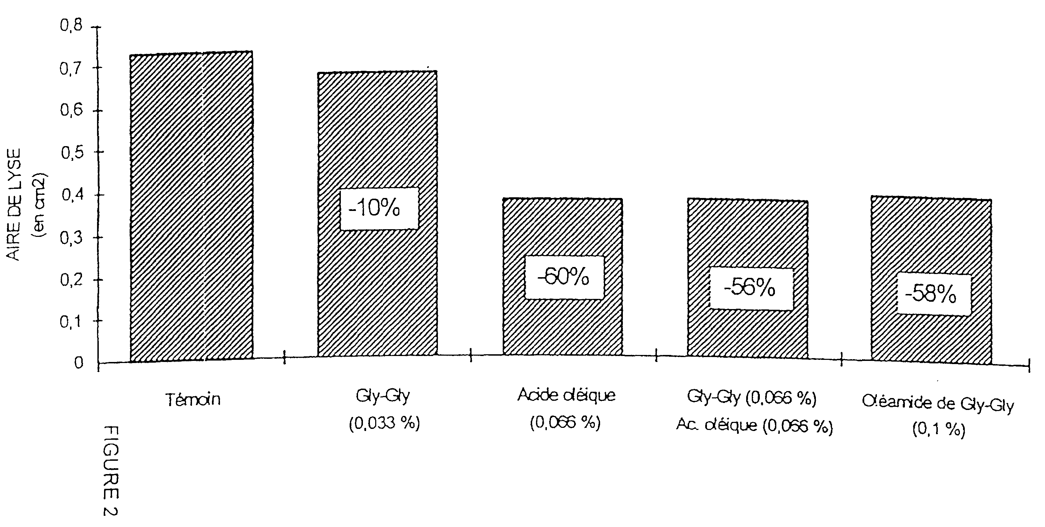

La figure 2 montre que l'oléamide de glycylglycine a une activité anti-élastase importante et que celle-ci est apportée par l'acide oléïque. En effet, le mélange acide oléique / glycylglycine a le même niveau d'activité que l'acide oléique.

### EXEMPLE 4 : Cinétique d'action de l'oléamine de glycylglycine sur le métabolisme cellulaire.

Pour objectiver cette activité, on utilise le modèle cellulaire de derme équivalent de type Bell. Il permet, grâce à une mise en culture de fibroblastes sur un support de collagène, de reproduire in vitro une structure tissulaire tridimensionnelle et de rendre compte des interactions fibroblaste-matrice, le fibroblaste étant impliqué dans le maintien de la structure dermique.

La capacité des fibroblastes, ensemencés dans un milieu défini avec ou sans actif, à organiser les fibres de collagène les unes par rapport aux autres et de les faire se rétracter, se concrétise par une diminution de surface de ce derme. Ce test traduit la capacité du fibroblaste à modeler, par ce biais, un véritable tissu. On suit donc la cinétique de contraction de ce derme sur 7 jours, cette cinétique étant liée au métabolisme de la cellule.

Les concentrations en actifs employées correspondent à des doses auxquelles ces actifs se sont avérés non cytotoxiques et non prolifératifs.

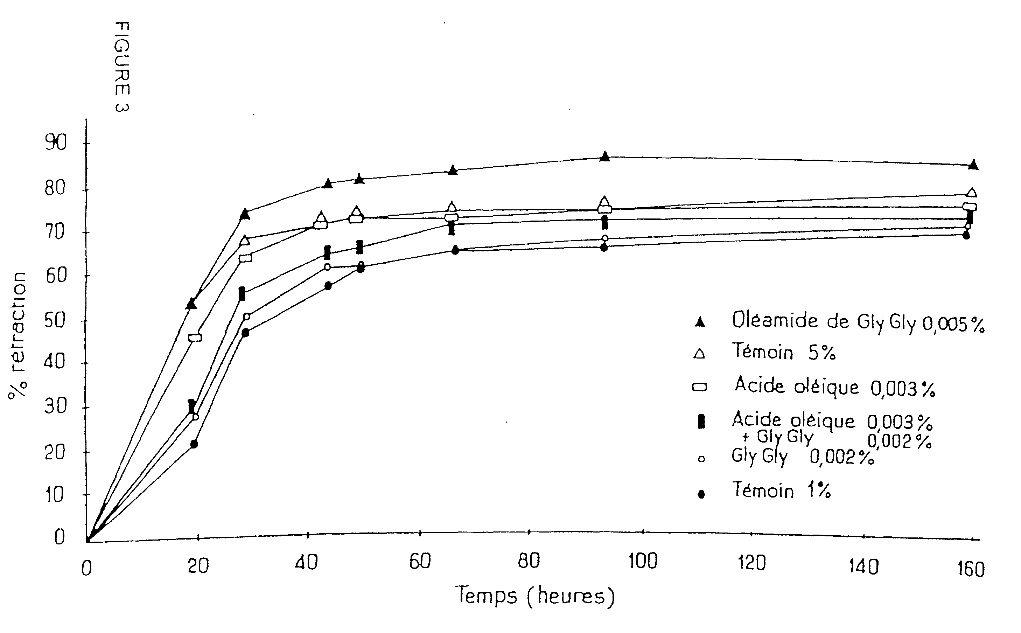

La figure 3 montre par ordre croissant d'activité que l'oléamide de glycylglycine est plus efficace que l'acide oléique, l'association acide oléique/glycylglycine, la glycylglycine.

Cela traduit l'activité stimulante très spécifique de l'oléamide de glycylglycine sur le métabolisme du fibroblaste (les deux actifs associés ayant peu d'activité) et donc sa capacité à régénérer un tissu.

Ainsi, l'oléamide de glycylglycine possède, en plus des propriétés de protection des fibres du tissu conjonctif, une activité propre sur le métabolisme du fibroblaste et, par là même, sur le vieillissement intrinsèque.

Les formules présentées dans les exemples suivants sont données à titre illustratif. Elles peuvent être généralisées à l'ensemble des catégories de produits de dermo-cosmétologie, de compléments par voie orale et de produits pharmaceutiques.

Dans les exemples de compositions, les chiffres sont exprimés en pourcentage massique.

### EXEMPLE 5 : Produit antisolaire avec filtres organiques et écrans minéraux :

| **1. PHASE HUILEUSE** | |
|---|---|
| ISODODECANE | 1 - 10 |
| TRIGLYCERIDE CAPRIQUE / CMRYLIQUE | 1 - 10 |
| PEG 45/ DODECYL GLYCOL COPOLYMER | 0,01 / 5 |
| | |

| **2. AGENT LIPOPHILE:** | |
|---|---|
| TRIGLYCERIDES DE LAIT HYDROXYLES | 0,1- 5 |
| | |

| **3. PHASE AQUEUSE** | |
|---|---|
| PEMULEN TR 1® | 0,01 - 0,09 |
| GOMME XANTHANE | 0,05 - 1 |
| | |

| **4. ACTIFS ET EXCIPIENTS** | |
|---|---|
| OLEAMIDE DE GLYCYLGLYCINE | 0,0001 - 20 |
| DIOXYDE DE TITANE ULTRA FIN | 1 - 30 |
| OXYDE DE ZINC ULTRA FIN | 1 - 30 |
| ESTERS CINNAMIQUES | 0,1 - 10 |
| DERIVES DU DIBENZOYL METHANE | 0,05 - 5 |
| CONSERVATEURS | QS |
| ANTI RADICALAIRES | 0,01 - 3 |
| PARFUM | QS |
| SODIUM HYDROXYDE | QS |
| EAU PURIFIEE QSP | 100 |

### EXEMPLE 6 : Baume après rasage

| **1. PHASE HUILEUSE** | |
|---|---|
| ISODODECANE | 1 - 20 |
| DIMETHICONE | 1 - 5 |
| | |

| **2. AGENT LIPOPHILE** | |
|---|---|
| TRISTEARATE DE SORBITAN | 0,01 - 5 |
| | |

| **3. PHASE AQUEUSE** | |
|---|---|
| ETHANOL à 95° DENATURE | 1 - 40 |
| BUTYLENE GLYCOL | 0,5 - 10 |
| MACROGOL 200 | 0,1 - 10 |
| POLYMERE RETICULE D'ALKYLACRYLATE EN C10-C30 | 0,05 - 2 |
| | |

| **4. ACTIFS ET EXCIPIENTS** | |
|---|---|
| OLEAMIDE DE GLYCYLGLYCINE | 0,0001 - 20 |
| LACTATE DE MENTHYLE | 0,1 - 1 |
| CHELATEUR DE FER | 0,001 - 5 |
| ALLANTOINE | 0,05 - 0,5 |
| ALPHA BISABOLOL | 0,1 - 1 |
| HYDRATANTS | 0,1 - 10 |
| D-PANTHENOL | 0,01 - 1 |
| NEUTRALTSANTS | QS |
| CONSERVATEURS | QS |
| COLORANTS | QS |
| PARFUM | QS |
| EAU PURIFEE QSP | 100 |

### EXEMPLE 7 : Lait corporel vitaminé :

| **1. PHASE HUILEUSE** | |
|---|---|
| BEURRE DE KARITE | 1 - 10 |
| TRIGLYCERIDE CAPRIQUE / CAPRYLIQUE | 1 - 10 |
| SQUALANE VEGETAL | 1 - 20 |
| EXTRAIT HUILEUX DE CALENDULA | 1 - 25 |
| | |

| **2. AGENT LIPOPHILE** | |
|---|---|
| TRIOLEATE DE SORBITAN | 0,05 - 4 |
| | |

| **3. PHASE AQUEUSE** | |
|---|---|
| PEMULEN TR 2 ® | 0,1 - 2 |
| VEEGUM ULTRA ® | 0,1 - 2 |
| DERIVE CELLULOSIQUE | 0,05 - 0,5 |
| GLYCEROL | 0,5 - 15 |
| BUTYLENE GLYCOL | 0,5 - 20 |
| | |

| **4. ACTIFS ET EXCIPIENTS** | |
|---|---|
| OLEAMIDE DE GLYCYLGLYCINE | 0,0001 - 20 |
| α-TOCOPHEROL | 0,01 |
| HYDROLYSAT DE PROTEINES | 0,001 - 5 |
| VITAMINE A PALMITATE | 0,01 - 0,5 |
| ACIDE ASCORBIQUE | 0,01 |
| ESTER GLYCERIQUE DE VITAMINE F | 0,5 |
| ALLANTOINE | 0,1 - 0,5 |
| CONSERVATEURS | QS |
| NEUTRALISANTS | QS |
| COLORANTS | QS |
| EAU THERMALE D'AVENE QSP | 100 |

### EXEMPLE 8 : Produit antisolaire 100 % minéral

| **1. PHASE HUILEUSE** | |
|---|---|
| | |
| C12-15 ALKYL BENZONATE | 1 - 10 |
| ISODODECANE | 1 - 20 |
| CYCLOMETHICONE | 1 - 20 |
| DIMETHICONE | 0,5 - 10 |
| | |

| **2. AGENT LIPOPHILE** | |
|---|---|
| TRIGLYCERIDES DE LAIT HYDROXYLES | 0,1 - 5 |
| | |

| **3. PHASE AQUEUSE** | |
|---|---|
| PEMULEN TR 1 ® | 0,05 - 3 |
| CARBOMER | 0,05 - 2 |
| | |

| **4. ACTIFS ET EXCIPIENTS** | |
|---|---|
| OLEAMIDE DE GLYCYLGLYCINE | 0,0001 - 20 |
| DIOXYDE DE TITANE ULTRA FIN | 1 - 30 |
| HYDROLYSAT DE PROTEINES | 0,001 - 5 |
| OXYDE DE ZINC ULTRA FIN | 0,1 - 30 |
| VITAMINE E ACETATE | 0,05 - 2 |
| SILICE HYDROGENEE | 0,05 - 2 |
| OXYDE DE FER ULTRA FIN | QS |
| CONSERVATEURS | QS |
| HYDROXYDE DE SODIUM | QS |
| PARFUM | QS |
| EAU THERMALE D'AVENE QSP | 100 |

### EXEMPLE 9 : Après soleil visage :

| **1. PHASE HUILEUSE** | |
|---|---|
| BEURRE DE KARITE | 1 - 10 |
| VASELINE BLANCHE | 1 - 15 |
| C12-15 ALKYL BENZONATE | 1 - 20 |

| **2. AGENT LIPOPHILE** | |
|---|---|
| TRIOLEATE DE SORBITAN | 0,5 - 3 |
| | |

| **3. PHASE AQUEUSE** | |
|---|---|
| GOMME XANTHANE | 0,1 - 0,5 |
| GLYCEROL | 5 - 10 |
| MACROGOL R 600® | 1 - 10 |
| OCTOXYGLYCERINE | 0,1 - 5 |
| | |

| **4. ACTIFS ET EXCIPIENTS** | |
|---|---|
| OLEAMIDE DE GLYCYLGLYCINE | 0,0001 - 20 |
| ALLANTOINE | 0,05 - 1 |
| AGENT CHELATEUR | 0,001 - 5 |
| ALPHA BISABOLOL | 0,05 - 1 |
| VITAMINE C | 0,01- 0,5 |
| VITAMINE E | 0,05 - 3 |
| VITAMINE E ACETATE | 0,1 - 2 |
| VITAMINE F ESTER GLYCERIQUE | 0,5 - 2 |
| CONSERVATEURS | QS |
| ANTIOXYDANTS | QS |
| COLORANTS | QS |
| PARFUM | QS |
| EAU THERMALE QSP | 100 |

### EXEMPLE 10 : Stick écran minéral 50B/10A :

| | |
|---|---|
| OLEAMIDE DE GLYCYLGLYCINE | 0,0001 - 20 |
| DOW CORNING 3225 C® | 10 |
| DECAMETHYL CYCLOPENTASILOXANE | 1 - 10 |
| PATE DE TITANE | 1 - 50 |
| SPECTRAVEIL MOTG® | 0,1 - 20 |
| VASELINE BLANCHE QUALITE A | 1,000 |
| BENTONE 38® STERILISEE | 0,1 - 2 |
| MONOSTEARATE DE GLYCEROL | 0,1 - 2 |
| MONOMULS 90L 12® | 0,1 - 2 |
| ACETATE D'ALPHA-TOCOPHEROL | 0,500 |
| NaCl SUPER EPURE DESULFATE | 2,000 |
| LELTROL TF | 0,150 |
| WITCONOL APM® | 0,800 |
| PARAHYDROXY BENZOATE | 0,600 |
| EUXYL K 400® | 0,100 |
| GLYCEROL | 8,000 |
| EDETATE DISODIQUE PH EU | 0,200 |
| MELANGE PIGMENTAIRE 14123 | 2,000 |
| PARFUM | 0,1000 |
| AEROSIL R972® | 0,100 |
| BUTYL HYDROXY TOLUENE | 0,0100 |
| EAU THERMALE D'AVENE | 100,00 |

### EXEMPLE 11 : Crème solaire minérale :

| | |
|---|---|
| OLEAMIDE DE GLYCYLGLYCINE | 0,0001 - 20 |
| CHELATEUR DE FER | 0,001 - 5 |
| ELFACOS S 37® | 0,6 |
| ABIL WE 09® | 4 |
| PARAFFINE LIQUIDE | 1 - 10 |
| TiO2 PATE | 1 - 70 |
| NaCI | 0,7 |
| CONSERVATEURS | QSP |
| OXYDE DE ZINC ENROBE | 1 - 5 |
| GUAR HYDROXYPROPYLTRIMONIUM | 0,7 |
| EAU QSP | 100 |
| BENTONE 38 - QUATERNIUM 18® | 0,1 - 5 |
| AEROSIL R 972® | 0,1 - 5 |

### EXEMPLE 12 : Crème solaire E/H minérale +/- organique :

| | |
|---|---|
| OLEAMIDE DE GLYCYLGLYCINE | 0,0001 - 20 |
| HOSTERACIN W/O | 10 - 15 |
| PATE DE TI02 | 1 - 70 |
| ANTARON WP 660 | 0,1 - 3 |
| ZnO ENROBE ET EMPATE A 50 % | 1 - 50 |
| CONSERVATEURS | QSP |
| CINNAMATE | 0 - 10 |
| DIBENZOYLMETHANE | 0 - 4 |
| EAU QSP | 100 |
| GAMMA ORYZANOL | 0,2 |
| BENTONE 38® | 0,1 - 5 |
| AEROSIL R 972® | 0,1 - 6 |

### EXEMPLE 13 : Crème solaire E/silicone :

| | |
|---|---|
| OLEAMIDE DE GLYCYLGLYCINE | 0,0001 - 20 |
| ABIL EM 90 | 1 - 5 |
| HYDROLYSAT DE PROTEINES | 0,001 - 5 |
| ABIL WAX 9801 | 0,2 - 6 |
| DOW 345 | 0,1 - 10 |
| TiO2 EMPATE A 30 % | 0,1 - 70 |
| ZnO ENROBE POUDRE | 0,1 - 25 |
| EAU QSP | 100 |
| NaCl | 0,1 - 3 |

### EXEMPLE 14 : Crème solaire E/H :

| | |
|---|---|
| OLEAMIDE DE GLYCYLCLYCINE | 0,0001 - 20 |
| ISOLAN GI 34® | 0,5 - 5 |
| AGENT CHELATEUR | 0,001 - 5 |
| TiO2 EMPATE A 50 % | 1 - 601 - 60 |
| ZnO EN SUSPENSION A 60 % | 1 - 50 |
| DOW 345 | 1 - 10 |
| VASELINE BLANCHE | 1 - 10 |
| EAU QSP | 100 |
| GLYCEROL | 1 - 10 |
| NaCI | 0,1 - 2 |

### EXEMPLE 15 : Crème solaire E/H silicone compact :

| | |
|---|---|
| OLEAMIDE DE GLYCYLGLYCINE ABIL WE 09® | 0,0001 - 20 |
| HYDROLYSAT DE PROTEINES | 0,5 - 5 |
| EAU | 0,001 - 5 |
| CIRE DE CARNAUBA | 1 |
| LANOLINE HYDROGENEE | 1 - 10 |
| ACETOXYSTEARATE | 1 - 10 |
| TiO2 EMPATE à 50 % dans le FINSOLV TN et le CETIOL V | 1 - 10 |
| ZnO ENROBE | 1 - 70 |
| BENZYLIDENE CAMPHRE | 1 - 25 |
| DIBENZOYL METHANE | 0 - 8 |
| BUTYL HYDROXY TOLUENE | 0 - 4 |
| OXYDE DE FER ENROBE | 10 - 2 |
| BENTONE 34® | 0,1 - 5 |
| AEROSIL® | 0,1 - 5 |

### EXEMPLE 16 : Fond de teint sans émulsionnant :

| | |
|---|---|
| OLEAMIDE DE GLYCYLGLYCINE | 0,0001 - 20 |
| TRIOLEATE DE SORBITAN | 0,1 - 2 |
| NYLON | 0 - 5 |
| LAUROYL LYSINE | 0 - 10 |
| TALC | 1 - 10 |
| DIOXYDE DE TITANE | 1 - 10 |
| Cl 77492® | 0,01 - 2 |
| Cl 77491® | 0,01 - 2 |
| Cl 77499® | 0,01 - 1 |
| ESTERS | 1 - 25 |
| CONSERVATEURS | QSP |
| PEMULEN TR1® | 0,01 - 1 |
| CARBOPOL 940® | 0 - 1 |
| VEEGUM | 0 - 1 |
| EAU | QSP |
| TEA pH | ≅ 6,5 |

### EXEMPLE 17 : Crème solaire

| | |
|---|---|
| OLEAMIDE DE GLYCYLGLYCINE | 0,0001 - 20 |
| DIBENZOYLMETHANE | 0,1 - 5 |
| OCTYL METHOXYCINNAMATE | 0,1 - 10 |
| DIOXYDE DE TITANE | 0,1 - 25 |
| PARAFFINE ET DIOXYDE DE TITANE ET OXYDE DE FER ET METHICONE | 0 - 15 |
| ALKYLBENZOATE EN C12-C15 | 0,5 - 15 |
| OCTYLDODECYL NEOPENTANOATE | 0,5 - 15 |
| MONOI | 0 - 5 |
| ALCOOL CETYLIQUE | 0 - 1 |
| CYCLOMETHICONE ET DIMETHICONOL | 0 - 10 |
| TRIOLEATE DE SORBITAN | 0,1 - 5 |
| ACETATE DE TOCOPHEROL | 0 - 1 |
| EAU OU EAU D'EAU THERMALE AVENE | QSP |
| GLYCERINE | 0 - 10 |
| POLYMERE RETICULE ACRYLATES/ ALKYLACRYLATES EN C10-C30 | 0,01 - 1 |
| CARBOMERE | 0 - 1 |
| HYDROXYPROPYLMETHYLCELLULOSE | 0 - 1 |
| CONSERVATEURS | QSP |
| EDTA DISODIQUE | 0 - 0,3 |
| PARFUM TRIETHANOLAMINE QSP | 6 - 8 |
| CAPTEUR DE RADICAUX LIBRES (Flavonoïdes, Extrait d'Hibiscus, Carotène) | 0 - 2 |

### EXEMPLE 18 : Crème solaire sans filtre chimique :

| | |
|---|---|
| OLEAMIDE DE GLYCYLGLYCINE | 0,0001 - 20 |
| TALC | 0 - 10 |
| AGENT CHELATEUR DU FER | 0,001 - 5 |
| DIOXYDE DE TITANE | 0,5 - 25 |
| OXYDE DE ZINC | 0,5 - 10 |
| SULFATE DE BARYUM | 0 - 5 |
| POLYGLYCERYL-10 DECASTEARATE | 0 - 5 |
| HUILE VEGETALE | 1 - 20 |
| HUILE MINERALE | 1 - 20 |
| POLYMERE RETICULE ACRYLATE / ALKYLACRYLATE EN C10-C30 | 0,01 - 1 |
| CARBOMERE | 0 - 1 |
| GOMME XANTHANE | 0 - 1 |
| EAU | QSP |
| CONSERVATEURS | QSP |
| TEA ou AMP pH | 6 - 8 |

### EXEMPLE 19 : Spray solaire

| | |
|---|---|
| OLEAMIDE DE GLYCYLGLYCINE | 0,0001 - 20 |
| CINNAMATE | 0 - 10 |
| HYDROLYSAT DE PROTEINES | 0,001 - 5 |
| SUSPENSION DE DIOXYDE DE TITANE | 0,5 - 20 |
| ALKYLBENZOATE EN C12-C15 | 0 - 10 |
| OCTYLDODECYL NOPENTANOATE | 0 - 10 |
| TRIOLEATE DE SORBITAN | 0,1 - 5 |
| HUILE MINERALE | 1 - 10 |
| CONSERVATEURS | QSP |
| EAU | QSP |
| PEMULEN TR2® | 0,01 - 1 |
| GOMME XANTHANE | 0 - 1 |
| TEA pH | 6 - 8 |

### EXEMPLE 20 : Lotion anti-chute :

| | |
|---|---|
| OLEAMIDE DE GLYCYLGLYCINE | 0,0001 - 20 |
| PROVITAMINE B5 | 0,3 |
| PYRIDOXINE CHLORHYDRATE | 0,3 |
| EXTRAIT HYDRO ALCOOLIQUE DE QUINQUINA | 1 |
| EXTRAIT FLUIDE DE CRESSON | 0,75 |
| EAU DE MENTHE POIVREE | 5 |
| PIROCTONOLAMINE | 0,05 |
| SULFATE DE ZINC | 0,10 |
| HUILE ESSENTIELLE DE ROMARIN | 0,05 |
| ALCOOL ETHYLIQUE | 23 (vol.) |
| ALCOOL LAURIQUE ETHOXYLE | 1,5 |
| EAU DEMINERALISEE QSP | 100 |

### EXEMPLE 21 : Mousse à raser :

| | |
|---|---|
| OLEAMIDE DE GLYCYLGLYCINE | 0,0001 - 20 |
| LANOLINE ACETYLEE | 0,5 |
| ACIDE STEARIQUE | 7 |
| POTASSE HYDROXIDE | 0,42 |
| TRI ETHANOLAMINE | 2,5 |
| ALCOOL GRAS SUPERIEUR ETHOXYLE | 3 |
| GLYCEROL | 5 |
| ALKANOLAMIDE D'ACIDES GRAS | 0,5 |
| EXTRAIT D'ALOE VERA POUDRE | 0,05 |
| D-PANTHENOL | 0,5 |
| EAU DEMINERALISEE | 100 |

### EXEMPLE 22 : Shampooing :

| | |
|---|---|
| OLEAMIDE DE GLYCYLGLYCINE | 0,0001 - 20 |
| TEA COCO HYDROXYLED ANIMAL PROTEINE (40%) | 7 |
| HYDROLYSAT DE PROTEINES DE BLE | 0,001 |
| LAURYL SULFATE D'AMMONIUM (28 %) | 8 |
| LAURYL ETHER SULFATE DE SODIUM (28 %) | 17 |
| BASE NACRANTE (20 %) | 10 |
| D-PANTHENOL | 0,5 |
| EDTA 2Na | 0,20 |
| HYDROXYPROPYL GUAR | 0,30 |
| DMDM HYDANTOINE | 0,20 |
| DETHANOLAMIDE D'ACIDES GRAS DE COPRAH | 2,6 |
| ACIDE CITRIQUE pH | 6,5 |
| PARFUM | QS |
| EAU DEMINERALISEE | 100 |

### EXEMPLE 23 : Gel douche :

| | |
|---|---|
| OLEAMIDE DE GLYCYLGLYCINE | 0,0001 - 20 |
| HYDROLYSAT DE PROTEINES | 0,001 - 5 |
| LAURETHSULFATE DE SODIUM | 20 |
| LAUROYL SARCOSINATE DE SODIUM | 15 |
| COCAMIDOPROPYLBETAINE | 5 |
| ACIDE MYRISTIQUE | 5 |
| OCTYLDODECANOL | 10 |
| COCOATE DE GLYCERYLE HEPTAETHOXYLE | 5 |
| TRIETHANOLAMINE | 0,25 |
| MONSTEARATE DE GLYCEROL | 1 |
| ISOHEXADECANE | 5 |
| LAURAMIDE DETHANOLAMINE | 4 |
| GLYCEROL | 1 |
| AUTRES ADDITIFS | 0,75 |
| EAU | 28 |

### EXEMPLE 24 : Sérum dépigmentant / anti-radicalaire / Kératolytique :

| | |
|---|---|
| OLEAMIDE DE GLYCYLGLYCINE | 0,0001 - 20 |
| HYDROLYSAT DE PROTEINES | 0,001 - 5 |
| EXTRAIT DE PILOSELLE | 0,1 - 10 |
| ACIDE SALYCILIQUE | 0,1 - 5 |
| VITAMINE C PCA | 0,1 - 10 |
| HYDROQUINONE | 0,1 - 5 |
| ALCOOL BATYLIQUE | 0,05 - 1 |
| ALCOOL A 95° | 10 - 75 |
| ADIPATE D'ISOPROPYLE | 5 - 15 |
| ACIDE OLEIQUE | 0,01 - 1 |
| PROPYLENE GLYCOL | 5 - 40 |
| KLUCEL MF | 0,1 - 2 |
| EAU QSP | 100 |

### EXEMPLE 25 : Gélule voie orale :

| | |
|---|---|
| LACTOSE QSP | 100 |
| AMIDON DE BLE | 5 |
| TALC | 3 |
| STEARATE DE MAGNESIUM | 0,5 |
| HYDROLYSAT DE PROTEINES | 0,001 - 5 |
| OLEAMIDE DE GLYCYLGLYCINE | 0,0001 - 20 |

### EXEMPLE 26 : Masque :

| | |
|---|---|
| EAU DEMINERALISEE QSP | 100 |
| GLYCERINE | 15 |
| KAOLIN | 15 |
| BUTYENE GLYCOL | 7 |
| HECTORITE | 4 |
| GLYCERYL STEARATE | 3 |
| PEG 100 STEARATE | 3 |
| DIMETHICONE | 2 |
| HUILE DE RICIN HYDROGENEE ETHOXYLEE | 1 |
| DIOXYDE DE TITANE | 1 |
| PARFUM | 0,1 |
| TRIETHANOLAMINE | 0,3 |
| OLEAMIDE DE GLYCYLGLYCINE | 0,0001 - 20 |
| HYDROLYSAT DE PROTEINES | 0,001 - 5 |

### EXEMPLE 27 : Emulsion visage :

| | |
|---|---|
| EAU DEMINERALISEE QSP | 100 |
| HUILE MINERALE | 5 |
| HUILE DE RICIN HYGROGENEE ETHOXYLEE | 153 |
| TRIETHANOLAMINE | 71,5 |
| DIMETHICONE | 41 |
| CARBOMER | 31 |
| PARFUM | 30,2 |
| OLEAMIDE DE GLYCYLGLYCINE | 0,0001 - 20 |
| AGENT CHELATEUR | 0,001 - 5 |

### EXEMPLE 28 : Pain dermatologique :

| | |
|---|---|
| OLEAMIDE DE GLYCYLGLYCINE | 0,0001 - 20 |
| AMIDON DE MAIS | 25 |
| LAURYL SULFATE DE POTASSIUM | 20 |
| ALCOOL CETEARYLIQUE | 14 |
| COCOYL ISETHIONATE DE SODIUM QSP | 100 |
| EAU DEMINERALISEE | 0,3 |
| PARFUM | |

### EXEMPLE 29 : Savon :

| | |
|---|---|
| SUIF QSP | 100 |
| COCOATE DE SODIUM | 19 |
| PARFUM | 1,5 |
| CIRE D'ABEILLE | 0,15 |
| ESTER DE CETYLE | 0,35 |
| OXYDE DE FER | 0,02 |
| DIOXYDE DE TITANE | 0,02 |
| OLEAMIDE DE GLYCYLGLYCINE | 0,0001 - 20 |

## Revendications

1. Utilisation de l'oléamide de glycylglycine pour fabriquer une composition destinée à lutter contre la glycation et l'élastose des fibres protéiques du tissu conjonctif.

2. Utilisation selon la revendication 1 pour lutter contre le vieillissement cutané photo-induit.

3. Utilisation selon la revendication 1 pour améliorer la qualité de la peau et des phanères.

4. Utilisation selon la revendication 1, pour lutter contre les complications du diabète.

5. Utilisation selon l'une des revendications précédentes, **caractérisé en ce que** la composition est une composition cosmétique, dermatologique ou alimentaire.

6. Utilisation selon la revendication 5, **caractérisée en ce que** la composition contient 0,0001 à 20 % en poids d'oléamide de glycylglycine par rapport au poids total de la composition.

7. Utilisation selon l'une des revendications 5 ou 6, **caractérisée en ce que** la composition contient, en outre, un agent anti-glyquant comme l'amino-guanidine, la glycylglycine ou le bromure de phénacylthiazolium.

8. Utilisation selon l'une des revendications précédentes, **caractérisée en ce qu'**elle contient en outre au moins un hydrolysat de protéine aux propriétés chélatrices.

9. Utilisation selon l'une des revendications précédentes, **caractérisée en ce qu'**elle contient un agent de protection contre les UV, en particulier un écran minéral.

## Patentansprüche

1. Verwendung von Glycylglycinoleamid zur Herstellung einer Zusammensetzung, die dazu bestimmt ist, die Glykierung und Elastose von Proteinfasern des Bindegewebes zu bekämpfen.

2. Verwendung nach Anspruch 1, um die Photo-induzierte Hautalterung zu bekämpfen.

3. Verwendung nach Anspruch 1, um die Qualität der Haut und der Phaneren zu verbessern.

4. Verwendung nach Anspruch 1, um die Komplikationen von Diabetes zu bekämpfen.

5. Verwendung nach einem der vorangehenden Ansprüche, **dadurch gekennzeichnet, dass** die Zusammensetzung eine kosmetische, dermatologische oder Nahrungsmittel-Zusammensetzung ist.

6. Verwendung nach Anspruch 5, **dadurch gekennzeichnet, dass** die Zusammensetzung 0,0001 bis 20 % Glycylglycinoleamid, bezogen auf das Gesamtgewicht der Zusammensetzung, enthält.

7. Verwendung nach einem der Ansprüche 5 oder 6, **dadurch gekennzeichnet, dass** die Zusammensetzung darüber hinaus ein Anti-Glykierungsmittel, wie Aminoguanidin, Glycylglycin oder Phenacylthiazoliumbromid, enthält.

8. Verwendung nach einem der vorangehenden Ansprüche, **dadurch gekennzeichnet, dass** sie darüber hinaus mindestens ein Protein-Hydrolysat mit chelatisierenden Eigenschaften enthält.

9. Verwendung nach einem der vorangehenden Ansprüche, **dadurch gekennzeichnet, dass** sie ein Schutzmittel gegen UV, insbesondere einen mineralischen Schutz, enthält.

## Claims

1. Use of glycylglycine oleamide to manufacture a composition intended for combating the glycation and elastosis of the protein fibers of connective tissue.

2. Use according to Claim 1, for combating photoageing of the skin.

3. Use according to Claim 1, for improving the quality of the skin and integuments.

4. Use according to Claim 1, for combating diabetes complications.

5. Use according to one of the preceding claims, **characterized in that** the composition is a cosmetic, dermatological or food composition.

6. Use according to Claim 5, **characterized in that** the composition contains 0.0001% to 20% by weight of glycylglycine oleamide relative to the total weight of the composition.

7. Use according to either of Claims 5 and 6, **characterized in that** the composition also contains an anti-glycating agent, for instance aminoguanidine, glycylglycine or phenacylthiazolium bromide.

8. Use according to one of the preceding claims, **characterized in that** it also contains at least one protein hydrolysate with chelating properties.

9. Use according to one of the preceding claims, **characterized in that** it contains an anti-UV agent, in particular a mineral sunblock.
